(19) Europäisches Patentamt
European Patent Office
Office européen des brevets

(11) Veröffentlichungsnummer: 0 331 636
A1

(12) **EUROPÄISCHE PATENTANMELDUNG**

(21) Anmeldenummer: 89810135.7

(22) Anmeldetag: 21.02.89

(51) Int. Cl.4: C 07 D 493/10
G 03 C 1/733, B 41 M 5/12,
B 41 M 5/14, B 41 M 5/22
//(C07D493/10,311:00,307:00)

(30) Priorität: 01.03.88 CH 763/88

(43) Veröffentlichungstag der Anmeldung:
06.09.89 Patentblatt 89/36

(84) Benannte Vertragsstaaten:
AT BE CH DE ES FR GB IT LI

(71) Anmelder: CIBA-GEIGY AG
Klybeckstrasse 141
CH-4002 Basel (CH)

(72) Erfinder: Zink, Rudolf
Alemannenstrasse 2
CH-4106 Therwil (CH)

(54) **2-Aralkylamino-Fluoranverbindungen, Verfahren zu deren Herstellung und deren Verwendung in Aufzeichnungsmaterialien.**

(57) Neue 2-Aralkylamino-Fluoranverbindungen der Formel

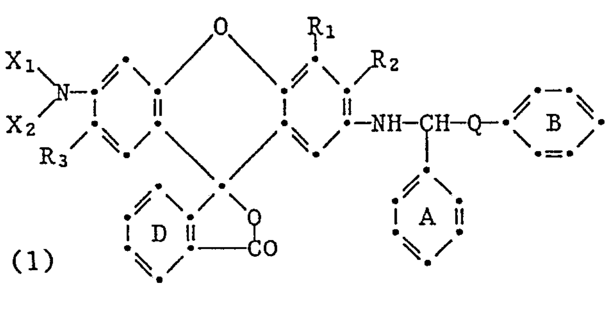

(1)

worin Q $-CH_2-$, $-CH_2-O-$ oder -CO-,
$R_1$, $R_2$ und $R_3$, unabhängig voneinander, je Wasserstoff, Halogen, Niederalkyl oder Niederalkoxy,
$X_1$ und $X_2$, unabhängig voneinander, je Wasserstoff, unsubstituiertes oder durch Halogen, Hydroxy, Cyano, Tetrahydrofuryl oder Niederalkoxy substituiertes Alkyl, Cycloalkyl oder unsubstituiertes oder durch Halogen, Cyano, Nitro, Niederalkyl, Niederalkoxy oder Niederalkoxycarbonyl substituiertes Benzyl oder Phenyl oder
$X_1$ und $X_2$ zusammen mit dem sie verbindenden Stickstoffatom einen fünf-oder sechsgliedrigen, heterocyclischen Rest bedeuten und worin die Benzolringe A und B, unabhängig voneinander, unsubstituiert oder durch Halogen, Cyano, Niederalkyl, Niederalkoxy, Niederalkylthio, Niederalkoxycarbonyl oder Trifluormethyl substituiert sind und der Ring D unsubstituiert oder durch Halogen, Nitro, Niederalkyl, Niederalkoxy, Niederalkylthio, Amino, Mononiederalkylamino oder Diniederalkylamino substituiert ist.

Diese Fluoranverbindungen eignen sich insbesondere als Farbbildner in druck- oder wärmeempfindlichen Aufzeichnungsmaterialien und ergeben intensive grüne, grünblaue, graue oder schwarze Farbtöne.

EP 0 331 636 A1

**Beschreibung**

**2-Aralkylamino-Fluoranverbindungen, Verfahren zu deren Herstellung und deren Verwendung in Aufzeichnungsmaterialien**

Die vorliegende Erfindung betrifft neue 2-Aralkylamino-Fluoranverbindungen, Verfahren zu ihrer Herstellung und ihre Verwendung als Farbbildner in druckempfindlichen oder wärmeempfindlichen Aufzeichungsmaterialien.

Die erfindungsgemässen 2-Aralkylamino-Fluoranverbindungen entsprechen der allgemeinen Formel

(1) 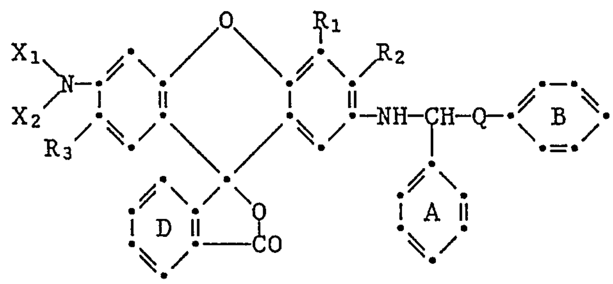

worin

Q $-CH_2-$, $-CH_2-O-$ oder $-CO-$,

$R_1$, $R_2$ und $R_3$, unabhängig voneinander, je Wasserstoff, Halogen, Niederalkyl oder Niederalkoxy,

$X_1$ und $X_2$, unabhängig voneinander, je Wasserstoff, unsubstituiertes oder durch Halogen, Hydroxy, Cyano, Tetrahydrofuryl oder Niederalkoxy substituiertes Alkyl mit höchstens 12 Kohlenstoffatomen, Cycloalkyl oder unsubstituiertes oder durch Halogen, Cyano, Nitro, Niederalkyl, Niederalkoxy oder Niederalkoxycarbonyl substituiertes Benzyl oder Phenyl oder

$X_1$ und $X_2$ zusammen mit dem sie verbindenden Stickstoffatom einen fünf-oder sechsgliedrigen, vorzugsweise gesättigten, heterocyclischen Rest bedeuten und worin

die Benzolringe A und B, unabhängig voneinander, unsubstituiert oder durch Halogen, Cyano, Niederalkyl, Niederalkoxy, Niederalkylthio, Niederalkoxycarbonyl oder Trifluormethyl substituiert sind und der Ring D unsubstituiert oder durch Halogen, Nitro, Niederalkyl, Niederalkoxy, Niederalkylthio, Amino, Mononiederalkylamino oder Diniederalkylamino substituiert ist.

Niederalkyl, Niederalkoxy und Niederalkylthio stellen bei der Definition der Reste der Fluoranverbindungen solche Gruppen oder Gruppenbestandteile dar, die 1 bis 5, insbesondere 1 bis 3 Kohlenstoffatome aufweisen. Beispiele für derartige Gruppen sind Methyl, Ethyl, n-Propyl, Isopropyl, n-Butyl, sek.-Butyl, tert.Butyl, Amyl, Isoamyl oder tert.Amyl bzw. Methoxy, Ethoxy, Isopropoxy, Isobutoxy oder tert.Butoxy bzw. Methylthio, Ethylthio, Propylthio oder Butylthio.

Halogen bedeutet beispielsweise Fluor, Brom oder vorzugsweise Chlor.

Q ist vorzugsweise $-CO-$ oder auch $-CH_2-$.

$R_1$, $R_2$ und $R_3$ sind vorzugsweise Wasserstoff, Methyl, Methoxy oder Chlor.

Stellen die Substituenten $X_1$ und $X_2$ Alkylgruppen dar, so können sie geradkettig oder verzweigt sein. Beispiele solcher Alkylreste sind Methyl, Ethyl, n-Propyl, Isopropyl, n-Butyl, sek.-Butyl, tert.-Butyl, 1,1,3,3-Tetramethylbutyl, Amyl, Isoamyl, n-Hexyl, 2-Ethyl-hexyl, n-Heptyl, n-Octyl, Isooctyl, n-Nonyl, Isononyl oder n-Dodecyl.

Sind die Alkylreste in $X_1$ und $X_2$ substituiert, so handelt es sich vor allem um Cyanoalkyl, Halogenalkyl, Hydroxyalkyl, Alkoxyalkyl jeweils vorzugsweise mit insgesamt 2 bis 6 Kohlenstoffatomen, wie z.B. β-Cyanoethyl, ß-Chlorethyl, γ-Chlorpropyl, ß-Hydroxyethyl, γ-Hydroxypropyl, ß-Methoxyethyl, ß-Ethoxyethyl oder γ-Methoxypropyl. Ein weiterer substituierter Alkylrest ist Tetrahydrofurfuryl.

Beispiele für Cycloalkyl in der Bedeutung der X-Reste sind Cyclopentyl, Cycloheptyl oder vorzugsweise Cyclohexyl. Die Cycloalkylreste können einen oder mehrere $C_1$-$C_4$-Alkylreste, vorzugsweise Methylgruppen, enthalten und weisen insgesamt 5 bis 10 Kohlenstoffatome auf.

Bevorzugte Substituenten in der Benzyl- und Phenylgruppe der X-Reste sind z.B. Halogen, Cyano, Methyl, Methoxy oder Carbomethoxy. Beispiele für derartige araliphatische bzw. aromatische Reste sind Methylbenzyl, 2,4-oder 2,5-Dimethylbenzyl, Chlorbenzyl, Dichlorbenzyl, Cyanobenzyl, Tolyl, Xylyl, 2,6-Dimethylphenyl, Chlorphenyl, Methoxyphenyl oder Carbomethoxyphenyl.

Wenn die Substituenten ($X_1$ und $X_2$) zusammen mit dem gemeinsamen Stickstoffatom einen heterocyclischen Rest darstellen, so ist dieser beispielsweise Pyrrolidino, Piperidino, Pipecolino, Morpholino, Thiomorpholino oder Piperazino, z.B. N-Methylpiperazino. Bevorzugte gesättigte heterocyclische Reste für $-NX_1X_2$ sind Pyrrolidino, Piperidino oder Morpholino.

Die Substituenten $X_1$ und $X_2$ sind vorzugsweise Cyclohexyl, Tolyl, Xylyl, Benzyl, Cyano-Niederalkyl z.B. ß-Cyanoethyl oder in erster Linie Niederalkyl, wie z.B. Methyl, Ethyl, n-Propyl, Isopropyl, n-Butyl oder Amyl.

$-NX_1X_2$ ist bevorzugt auch Pyrrolidinyl, Tetrahydrofurfurylamino oder N-Niederalkyl-N-tetrahydrofurfurylamino.

Die Benzolringe A und B sind vorzugsweise unsubstituiert. Als Substituenten können sie in bevorzugter Weise Methyl, Chlor, Cyano, Trifluormethyl, Methoxy oder Carbomethoxy enthalten.

Der Ring D ist ebenfalls vorzugsweise nicht weiter substituiert. Falls er Substituenten aufweist, ist er in erster Linie durch Halogen, Nitro oder Diniederalkylamino substituiert.

Praktisch wichtige 2-Aralkylamino-Fluoranverbindungen entsprechen der Formel

(2)

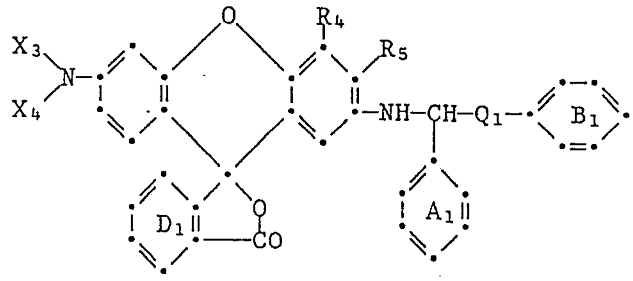

worin

$Q_1$ $-CH_2-$ oder vorzugsweise $-CO-$,

$R_4$ und $R_5$, unabhängig voneinander, je Wasserstoff, Halogen oder Niederalkyl,

$X_3$ $C_1$-$C_6$-Alkyl, $C_5$-$C_6$-Cycloalkyl, Benzyl, Phenyl oder durch Halogen, $C_1$-$C_4$-Alkyl oder $C_1$-$C_4$-Alkoxy substituiertes Phenyl, $X_4$ $C_1$-$C_6$-Alkyl oder Benzyl oder $-NX_3X_4$ Pyrrolidinyl, Piperidinyl, Morpholinyl oder auch Tetrahydrofurfurylamino oder N-Niederalkyl-N-tetrahydrofurfurylamino

bedeuten und worin

die Benzolringe $A_1$ und $B_1$, unabhängig voneinander, je unsubstituiert oder durch Halogen, Niederalkyl oder Niederalkoxy, substituiert sind und der Ring $D_1$ unsubstituiert oder durch Halogen, substituiert ist.

Unter den Verbindungen der Formel (2) sind die Fluoranverbindungen, in denen $X_3$ und $X_4$ $C_1$-$C_4$-Alkyl, $R_4$ und $R_5$ Wasserstoff, Methyl oder Chlor bedeuten und die Ringe $A_1$, $B_1$ und $D_1$ unsubstituiert sind, bevorzugt.

Von besonderem Interesse sind Fluoranverbindungen der Formel

(3)

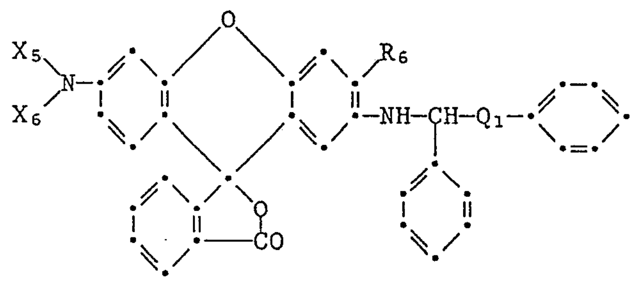

worin $Q_1$ $-CH_2-$ oder vorzugsweise $-CO-$,

$R_6$ Wasserstoff oder Methyl,

$X_5$ $C_1$-$C_4$-Alkyl, Cyclohexyl oder Tolyl

und $X_6$ $C_1$-$C_4$-Alkyl bedeuten.

Die erfindungsgemässen 2-Aralkylamino-Fluoranverbindungen der Formeln (1) bis (3) werden dadurch hergestellt, dass man eine Fluoranverbindung der Formel

(4)

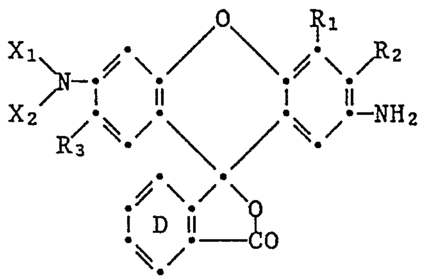

worin $R_1$, $R_2$, $R_3$, $X_1$, $X_2$ und D die angegebene Bedeutung haben, mit einem Aralkylierungsmittel der Formel

(5)

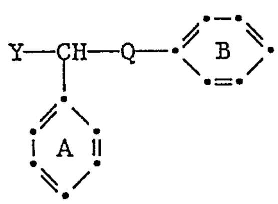

worin A, B und Q die angegebene Bedeutung haben und
Y Halogen, Alkylsulfonyloxy oder Arylsulfonyloxy bedeutet, umsetzt. Y ist vorzugsweise Chlor, Phenylsulfonyloxy, Bromphenylsulfonyloxy, Nitrophenylsulfonyloxy oder insbesondere p-Tolylsulfonyloxy.

Die als Ausgangsstoffe benötigten 2-Amino-Fluoranverbindungen der Formel (4) können auf bekannte Art und Weise durch Umsetzung einer Verbindung der Formel

(6)

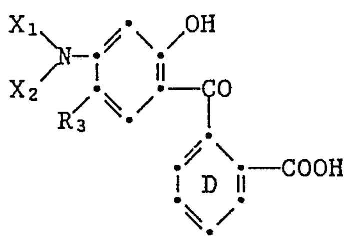

worin $X_1$, $X_2$, $R_3$ und D die angegebene Bedeutung haben, mit einem Aminophenolderivat der Formel

(7)

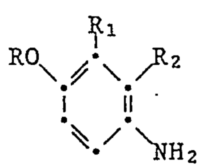

worin $R_1$ und $R_2$ die angegebene Bedeutung haben und R Wasserstoff oder Methyl bedeutet, in Gegenwart eines Kondensationsmittels hergestellt werden.

Die Umsetzung der Fluoranverbindung der Formel (4) mit dem Aralkylierungsmittel der Formel (5) erfolgt zweckmässigerweise bei erhöhter Temperatur, vorzugsweise bei 70°C bis 120°C und in Anwesenheit eines säurebindenden Mittels.

Geeignete säurebindende Mittel sind z.B. Alkalimetallhydroxide, Erdalkalimetallhydroxide, Alkalimetallhydrogencarbonate, Alkalimetallcarbonate oder tertiäre Stickstoffbasen, wie z.B. Pyridin, N-Methylpiperidin oder Trialkylamine oder auch Mischungen dieser Verbindungen. Bevorzugt sind Natriumhydrogencarbonat und Kaliumcarbonat.

Die Reaktion findet vorteilhafterweise in einem organischen Lösungsmittel, wie z.B. Methanol, Ethanol, Isopropanol, Methylcellosolve, Ethylcellosolve, Aceton, Methylethylketon, Methylisopropylketon, Dimethylformamid oder vorzugsweise in einem aromatischen Lösungsmittel, wie z.B. Benzol, Toluol, Xylol, Chlorbenzole wie Dichlorbenzol oder Trichlorbenzol, oder Nitrobenzol statt. Bevorzugte Lösungsmittel sind Toluol und Xylol.

Bevorzugte Aralkylierungsmittel der Formel (5) sind Desylchlorid, Desylbenzolsulfonat oder Desyl-p-Toluolsulfonat.

In einem Alternativverfahren können die 2-Aralkylamino-Fluoranverbindungen der Formeln (1) bis (3), in denen Q -CO- ist, dadurch hergestellt werden, dass man eine 2-Amino-Fluoranverbindung der Formel (4) mit einer Verbindung der Formel

(8) 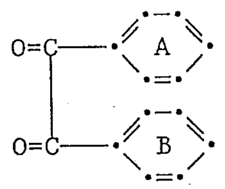

worin A und B die angegebene Bedeutung haben, umsetzt und das Umsetzungsprodukt der Formel

(9) 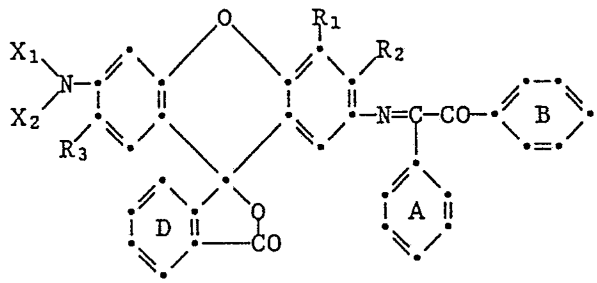

worin A, B, D, $R_1$, $R_2$, $R_3$, $X_1$ und $X_2$ die angegebene Bedeutung haben, reduziert.

Die Umsetzung der Fluoranverbindung der Formel (4) mit der Ketoverbindung der Formel (8) kann bei einer Temperatur von 10 bis 150°C vorgenommen werden. Als Reaktionsmedium kann ein organisches Lösungsmittel verwendet werden. Es ist vorteilhaft, einen sauren Katalysator zuzusetzen. Beispiele für derartige Katalysatoren sind niedere aliphatische Carbonsäuren oder deren Anhydride wie Essigsäureanhydrid sowie Zinkchlorid, Schwefelsäure, Phosphorsäure oder Phthalsäure.

Als Lösungsmittel kommen beispielsweise cycloaliphatische oder aromatische Kohlenwasserstoffe, wie z.B. Cyclohexan, Benzol, Toluol oder Xylol; Chlorkohlenwasserstoffe, wie Chloroform, Ethylenchlorid oder Chlorbenzol; Ether, wie Diethylether oder Glykoldimethylether; cyclische Ether, wie Dioxan oder Tetrahydrofuran; sowie Dimethylformamid, Diethylformamid, Dimethylsulfoxid, Essigsäure oder Acetonitril in Betracht.

Die Reduktion erfolgt zweckmässig in Ethern, wie Diethylether, Tetrahydrofuran oder Dioxan bei Temperaturen von 20 bis 120°C, vorzugsweise bei der Siedetemperatur des Lösungsmittels.

Als Reduktionsmittel eignen sich z.B. Metallhydride, wie Lithiumaluminiumhydrid oder Natriumborhydrid. Die Reduktion kann auch katalytisch mit Wasserstoff in Gegenwart von Metallkatalysatoren, wie z.B. Platin, Raney-Nickel oder Palladium-Kohle durchgeführt werden.

Ein weiteres Verfahren zur Herstellung der Fluoranverbindungen der Formeln (1) bis (3) besteht darin, dass man eine Fluoranverbindung der Formel (4) mit einer Carbinolverbindung der Formel

(10) 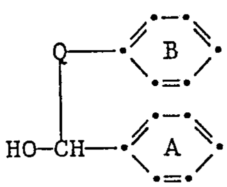

worin A, B und Q die angegebene Bedeutung haben, umsetzt.

Die Umsetzung der Fluoranverbindung der Formel (4) mit der Carbinolverbindung der Formel (10) erfolgt zweckmässig in einem polaren organischen Lösungsmittel, besonders in niederen aliphatischen Alkoholen, wie z.B. Methanol, Ethanol oder Isopropanol oder in Ethern, wie z.B. Tetrahydrofuran und vorzugsweise in

Anwesenheit eines sauren Katalysators. Die Kondensation kann schon bei Raumtemperatur (20 bis 25°C) vorgenommen werden. Zweckmässig ist jedoch die Anwendung einer erhöhten Temperatur bis zur Rückflusstemperatur, vorzugsweise bei 40 bis 100°C. Als saure Katalysatoren eignen sich anorganische Säuren, wie z.B. Salzsäure, Schwefelsäure, Phosphorsäure oder Perchlorsäure, sowie auch niedere aliphatische Carbonsäuren, wie Ameisensäure oder Essigsäure.

Die Verbindungen der Erfindung weisen mindestens zwei asymmetrische Kohlenstoffatome oder chirale Zentren auf und stellen diastereomere Gemische dar. Die einzelnen diastereomeren Komponenten (A und B) können durch Säulenchromatographie an Silicagel oder durch Kristallisation isoliert werden und lassen sich nach ihren physikalisch-chemischen Eigenschaften eindeutig unterscheiden. Als Elutionsmittel werden hauptsächlich Toluol, Essigsäureethylester, Methanol, Isopropanol, Chloroform und deren Gemische verwendet.

Die Fluoranverbindungen der Formeln (1) bis (3) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie je nach Bedeutung von Q und dem verwendeten Entwickler intensive violette, grüne, grün-blaue, graugrüne, graue oder schwarze Farbtöne, die besonders sublimationsecht sind.

Die Fluoranverbindungen der Formeln (1) bis (3) sind auch sehr wertvoll im Gemisch mit einem oder mehreren anderen bekannten Farbbildnern, z.B. 3,3-(Bis-aminophenyl-)-phthaliden, 3-Indolyl-3-aminophenylazaphthaliden, (3,3-Bis-indolyl-)-phthaliden, 3,6-Bisalkoxyfluoranen, 3-Aminofluoranen, 6-Dialkylamino-2-dibenzylaminofluoranen, 6-Dialkylamino-3-methyl-2-arylaminofluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen, um graue oder schwarze Färbungen zu ergeben.

Die Fluoranverbindungen der Formeln (1) bis (3) zeigen sowohl auf aktivierten Tonen, wie auch auf phenolischen Unterlagen eine ausgezeichnete Farbintensität. Sie eignen sich insbesondere als sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann. Sie zeichnen sich dadurch aus, dass sie pH stabil und in den Kapselölen gut löslich sind. Nach Belichtung in CB-Blatt weisen sie eine geringe Abnahme der Farbstärke (CB-Desaktivierung) auf.

Ein druckempfindliches Material besteht beispielsweise aus mindestens einem Paar von Blättern, die mindestens einen Farbbildner der Formeln (1) bis (3) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, Zinknitrat, Zirkondioxid, aktiviertes Kaolin oder irgendein beliebiger Ton. Als Entwickler können auch sauer reagierende, organische Verbindungen, wie z.B. gegebenenfalls ringsbustituierte Phenole, Resorcine, Salicylsäuren, wie z.B. 3,5-Bis-($\alpha$,$\alpha$-dimethylbenzyl-)-salicylsäure oder 3,5-Bis-($\alpha$-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein.

Die Entwickler können zusätzlich auch im Gemisch mit an sich unreaktiven oder wenig reaktiven Pigmenten oder weiteren Hilfstoffen wie Kieselgel oder UV-Absorbern, wie z.B. 2-(2′-Hydroxyphenyl-)benztriazolen eingesetzt werden. Beispiele für solche Pigmente sind: Talk, Titandioxid, Aluminiumoxid, Aluminiumhydroxyd, Zinkoxid, Kreide, Tone wie Kaolin, sowie organische Pigmente, z.B. Harnstoff-Formaldehydkondensate (BET-Oberfläche 2-75 $m^2/g$) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. halogeniertes Paraffin oder Diphenyl, wie Chlorparaffin, Monochlordiphenyl oder Trichlordiphenyl, ferner Tricresylphosphat, Di-n-butylphthalat, Dioctylphthalat, Trichlorbenzol, Trichlorethylphosphat, aromatische Ether, wie Benzylphenylether, Kohlenwasserstofföle, wie Paraffin oder Kerosin, z.B. mit Isopropyl, Isobutyl, sek-Butyl oder

tert.-Butyl alkylierte Derivate von Diphenyl, Naphthalin oder Terphenyl, Dibenzyltoluol, partiell hydriertes Terphenyl, mono- bis tetra-$C_1$-$C_3$-alkylierte Diphenylalkane, Dodecylbenzol, benzylierte Xylole, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Bei der Einkapselung zeichnen sich die erfindungsgemässen Fluoranverbindungen dadurch aus, dass sie ausserordentlich hohe pH-Beständigkeit z.B. in einem pH-Bereich von 4 bis 10 zeigen.

Die Kapselwände können durch Koazervationskräfte gleichmässig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (1) bis (3) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (1) bis (3) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel und/oder Wachs.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 12,51,348 beschrieben sind, z.B. 4-tert.-Butylphenol, 4-Phenylphenol, Methylen-bis-(p-phenylphenol), 4-Hydroxydiphenylether, α-Naphthol, ß-Naphthol, 4-Hydroxybenzoesäure-methylester oder -benzylester, 4-Hydroxydiphenylsulfon, 4′-Hydroxy-4-methyldiphenylsulfon, 2,4-Dihydroxydiphenylsulfon, 4′-Hydroxy-4-isopropoxydiphenylsulfon, 4-Hydroxyacetophenon, 2,2′-Dihydroxydiphenyl, 4,4′-Cyclohexylidendiphenol, 4,4′-Isopropylidendiphenol, 4,4′-Isopropyliden-bis-(2-methylphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Fluoranverbindungen und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem

Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Poly acrylsäure, Hydroxyethylcellulose, Methylcellulose, Carboxymethylcellulose, Polyacrylamid, Polyvinylpyrrolidon, carboxylierte Butadien-Styrol-copolymerisate, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumoxyd, Aluminiumhydroxyd, Calciumcarbonat (z.B. Kreide), Tone oder auch organische Pigmente, wie z.B. Harnstoff-Formaldehydpolymerisate enthalten. Um zu bewirken, dass nur innerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Bis-Stearoyl-ethylendiamid, Stearinsäureamid, Phthalsäureanhydrid, Metallstearate, wie z.B. Zinkstearat, Phthalsäurenitril, Dimethylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formeln (1) bis (3) ist die Herstellung eines Farbbildes mittels photohärtbarer Mikrokapseln, wie sie z.B. in der DE-OS 3 247 488 beschrieben sind.

In den folgenden Beispielen beziehen sich die angegebenen Prozentsätze, wenn nichts anderes angegeben ist, auf das Gewicht.

Beispiel 1:

(a) 11,6 g 2-Amino-6-diethylaminofluoran werden in 50 ml Toluol bei 25°C teilweise gelöst. Unter Rühren gibt man 6,3 g Benzil und 0,3 g Schwefelsäure (96 %) zu. Man hält das Reaktionsgemisch während 30 Stunden unter Rückfluss und entfernt das entstehende Wasser mittels Wasserabscheider. Hierauf wird das Reaktionsgemisch mit wässrigem Ammoniak neutralisiert. Alsdann wird die Toluolphase abgetrennt, aufkonzentriert und mit Isopropylalkohol versetzt, wobei ein Niederschlag gebildet wird. Dieser wird abfiltriert und getrocknet. Man erhält 15,6 g einer Verbindung der Formel

(i)

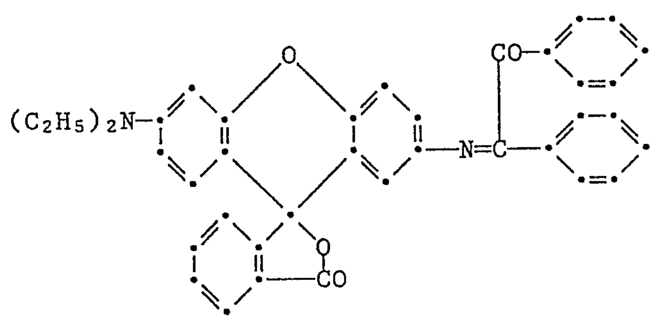

Nach Umkristallisation aus Toluol/Petrolether weist die Verbindung der Formel (i) einen Schmelzpunkt von 225-227°C auf. Auf Säureton entwickelt die Verbindung der Formel (i) sofort eine rote Färbung.

(b) 4,4 g der Verbindung der Formel (i) werden in 100 ml Dioxan mit 1,4 g Pt/C 5 % bei 15-20°C in 2 1/4 Stunden mit 214 ml Wasserstoff hydriert. Nach Entfernung des Katalysators und des Lösungsmittels und Trocknung des Produkts erhält man 4,1 g einer Fluoranverbindung der Formel

(11) 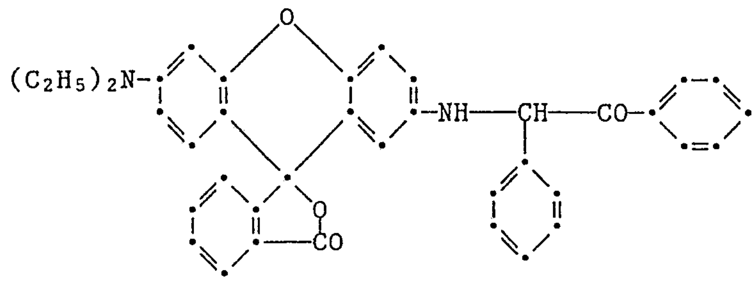

Diese Verbindung stellt ein diastereomeres Gemisch dar. Durch Säulenchromatographie an Kieselgel mit Toluol und 5 % Essigsäureethylester wird das diastereomere Gemisch in die einzelnen diastereomeren Komponenten A ($R_f$ 0,57) und B ($R_f$ 0,64) aufgetrennt.

Komponente A zeigt nach Umkristallisation aus Toluol/Isopropylalkohol einen Schmelzpunkt von 176-177° C. Auf säuremodifiziertem Ton entwickelt Komponente A sofort eine violett-graue Färbung. Auf Phenolharz-Papier ergibt sie eine grün-graue Färbung.

Komponente B zeigt nach Umkristallisation aus Toluol/Isopropylalkohol einen Schmelzpunkt von 169-171° C. Auf säuremodifiziertem Ton entwickelt Komponente B sofort eine rot-graue Färbung. Auf Phenolharz-Papier ist die Färbung grau.

Beispiel 2:

7,7 g 2-Amino-6-diethylaminofluoran werden in 30 ml Xylol mit 2,8 g Kaliumcarbonat und 7,3 g Desyl-p-Toluolsulfonat [J. Am. Chem. Soc. 73, (1951), 2635] während 18 Stunden bei 80° C gerührt. Das in der Reaktionslösung enthaltene diastereomere Gemisch der Formel (11) wird mittels Säulenchromatographie wie in Beispiel 1 beschrieben in die diastereomeren Komponenten A und B aufgetrennt.

Beispiel 3:

8,5 g 2-Amino-6-diethylaminofluoran werden unter Rühren in 100 ml Isopropylalkohol mit 4,2 g Benzoin und 0,2 g Schwefelsäure 96 % versetzt. Man hält das Gemisch während 4 Stunden unter Rückfluss, neutralisiert dann mit wässriger Ammoniaklösung und kühlt auf Raumtemperatur ab. Der Niederschlag wird abfiltriert, mit 100 ml 1N-Salzsäure bei 40-45° C behandelt, nochmals abfiltriert und in Toluol aufgenommen. Die Toluollösung wird mit wässriger Ammoniaklösung neutralisiert und von der Wasserphase getrennt. Die Toluollösung wird dann soweit aufkonzentriert bis vom diastereomeren Gemisch der Formel (11) die Komponente A auskristallisiert. Nach Reinigung mit Isopropylalkohol, Abfiltrieren und Trocknen erhält man 1,9 g der Komponente A gemäss Beispiel 1.

Komponente B (0,6 g) wird nach Säulenchromatographie des Filtrates gewonnen.

Beispiel 4:

7,7 g 2-Amino-6-diethylaminofluoran werden in 100 ml Ethylalkohol (95 %) unter Rühren mit 2,5 g Natriumhydrogencarbonat, 0,9 g Kaliumjodid und 6,9 g Desylchlorid versetzt. Man heizt das Gemisch auf Rückflusstemperatur und hält diese Temperatur während zwei Stunden. Bei Aufarbeitung des Reaktionsproduktes wie im Beispiel 3 beschrieben erhält man die Auftrennung des diastereomeren Gemisches der Formel (11) in die Komponenten A und B mit den in Beispiel 3 beschriebenen Eigenschaften.

Beispiel 5:

7,7 g 2-Amino-6-diethylaminofluoran werden in 25 ml Tetrahydrofuran bei 20-25° C mit 5,4 g Phthalsäure und 3,9 g Desoxybenzoin (Phenylbenzylketon) suspendiert. Hierauf werden zuerst 0,8 g Natriumborhydrid während 50 Minuten unter Rühren zugestreut und dann das Reaktionsgemisch während 1 1/2 Stunden bei 50° C gehalten. Danach werden 2 ml Essigsäure, 1 ml Wasser und 4,9 g Chloranil zugegeben, worauf die Reaktion 1 Stunde bei 30-40° C verläuft. Nach Zugabe von 100 ml Toluol und 100 ml Wasser werden die Phasen getrennt und die Toluolphase eingeengt. Man erhält 6,5 g einer Fluoranverbindung der Formel

(12) 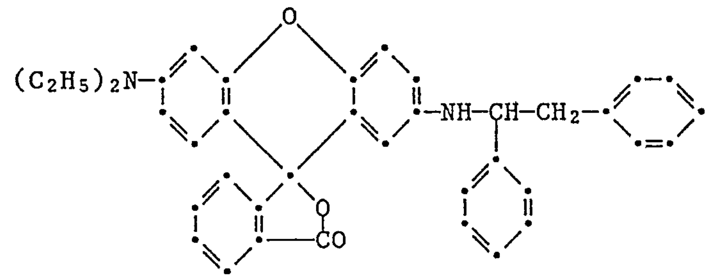

Diese Fluoranverbindung stellt ein diastereomeres Gemisch dar. Dieses Gemisch entwickelt auf säuremodifiziertem Ton sofort eine schwarze Färbung.

Im Dünnschichtchromatogramm auf Kieselgel mit Toluol/Essigsäureethylester (1:1) wird das diastereomere Gemisch in die Einzelkomponenten A ($R_f$ 0,61) und B ($R_f$ 0,68) getrennt. Nach Umkristallisieren in Isopropylalkohol erhält man 1,2 g der Komponente A, Smp. 175-176,5°C und 0,7 g der Komponente B, Smp. 227-229°C.

Komponente A entwickelt auf säuremodifiziertem Ton sofort eine graue, mit Phenolharz eine grau-grüne Färbung.

Komponente B entwickelt auf säuremodifiziertem Ton sofort eine graue, mit Phenolharz eine grün-graue Färbung.

Beispiel 6:

12 g 2-Amino-3-methyl-6-diethylaminofluoran werden in 150 ml Ethylalkohol (95 %) unter Rühren mit 3,8 g Natriumhydrogencarbonat, 1,3 g Kaliumjodid und 10,4 g Desylchlorid versetzt. Man heizt das Gemisch unter Stickstoff auf 55°C und hält diese Temperatur während 6 1/2 Stunden. Zur Isolierung des diastereomeren Gemisches wird das Reaktionsprodukt bei 20°C abfiltriert, mit Isopropylalkohol und Wasser gewaschen und bei 80°C im Vakuum getrocknet. Man erhält 9,5 g einer Fluoranverbindung der Formel

(13) 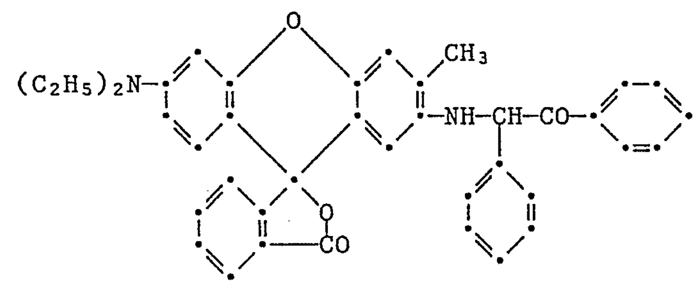

Diese Fluoranverbindung stellt ein diastereomeres Gemisch dar. Dieses Gemisch entwickelt auf säuremodifiziertem Ton sofort eine schwarze Färbung.

Zur Reinigung und Auftrennung des Gemisches wird das Rohprodukt in Toluol heiss gelöst, mit wässrigem Ammoniak extrahiert und das abgetrennte Toluol soweit aufkonzentriert, bis die Komponente A auskristallisiert und durch Filtration isoliert wird. Nach Trocknung erhält man 1,3 g der Komponente A mit einem Schmelzpunkt von 251-252°C.

Komponente A entwickelt mit Phenolharz sofort eine schwarze Farbe.

Zur Isolierung der Komponente B wird das oben erhaltene Filtrat durch Säulenchromatographie an Kieselgel mit Toluol und 20 % Essigsäureethylester aufgetrennt. Die Ausbeute an Komponente B beträgt 1,4 g, der Schmelzpunkt 146-148°C. Mit Phenolharz entwickelt Komponente B sofort eine schwarze Farbe.

Beispiel 7:

7,95 g 2-Amino-6-dibutylaminofluoran werden bei 50°C zu 20 g geschmolzenem Desoxybenzoin in 30 ml Tetrahydrofuran gegeben und mit 8,4 g Phthalsäure versetzt. Während 1 1/2 Stunden gibt man bei 50-55°C 1,45 g Natriumborhydrid und rührt das Reaktionsgemisch während 4 1/2 Stunden bei 60°C. Man verdünnt mit 30 ml Tetrahydrofuran, filtriert und engt das Filtrat zur Trockene ein. Der Rückstand wird bei 55°C in 150 ml Salzsäure (30 %) aufgenommen und mit 200 ml Toluol extrahiert. Die wässerige Phase wird mit Natriumhydroxidlösung (30 %) neutralisiert und mit Toluol extrahiert, worauf die Toluolphase zur Trockene eingeengt wird. Man erhält 11,6 g einer Fluoranverbindung der Formel

(14)

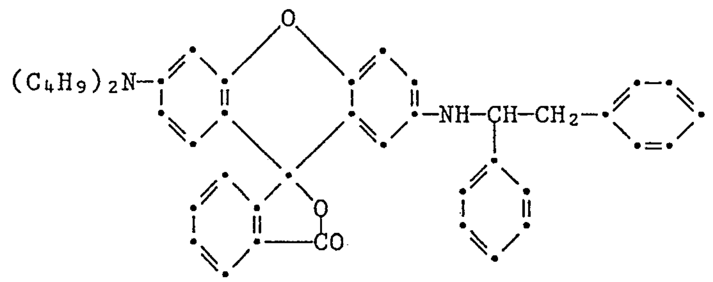

als diastereomeres Gemisch. Durch zweimalige Umkristallisation aus Isopropylalkohol mit 10 % Toluol erhält man 2,7 g der reinen Komponente A mit einem Schmelzpunkt von 205-206°C. Diese entwickelt mit Zinksalicylat sofort eine olive-graue Farbe.

Das oben angefallene Filtrat wird durch Säulenchromatographie an Kieselgel mit Toluol und 10 % Essigsäureethylester in die einzelnen Fraktionen getrennt. Man erhält so die Komponente B, nach Entfernung des Lösungsmittels, als amorphe Substanz von 1,4 g mit einem Erweichungspunkt bei 66°C.

Die Komponente B entwickelt auf Zinksalicylat sofort eine olive-graue Farbe.

Unter Verwendung von 2-Amino-3-methyl-6-diethylaminofluoran, 2-Amino-6-dibutylaminofluoran oder 2-Amino-3-methyl-6-dibutylaminofluoran werden gemäss den Beispielen 1 bis 7 die entsprechenden Fluoranverbindungen der Formel

(15)

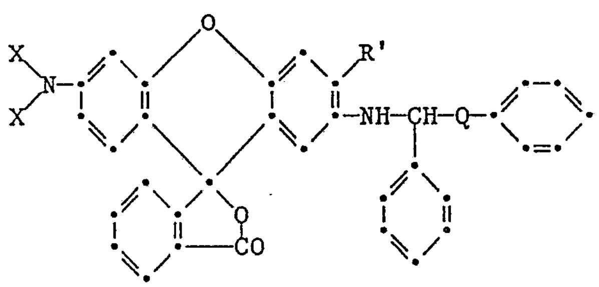

erhalten, welche diastereomere Gemische darstellen. Die aufgetrennten diastereomeren Komponenten A und B entwickeln auf sauren Ton die in der Tabelle angegebene Farbe.

Tabelle

| Beispiel | X | R′ | Q | Komponente A Farbe | Komponente B Fabre |
|---|---|---|---|---|---|
| 8 | $-C_2H_5$ | $-CH_3$ | $-CH_2-$ | grau | grau |
| 9 | $-n-C_4H_9$ | H | -CO- | olive | olive |
| 10 | $-n-C_4H_9$ | $-CH_3$ | -CO- | grau | grau |
| 11 | $-n-C_4H_9$ | $-CH_3$ | $-CH_2-$ | grau | grau |

Beispiel 12:

11,6 g 2-Amino-6-diethylaminofluoran werden in 20 ml Tetrahydrofuran bei 45-50°C mit 7,5 g Phthalsäure und 36 g ω-Phenoxyacetophenon versetzt. Bei 50-55°C werden über einen Zeitraum von 2 1/4 Stunden 1,25 g Natriumborhydrid in kleinen Portionen eingetragen. Man lässt das Reaktionsgemisch während 5 Stunden bei 58-60°C ausreagieren, gibt weitere 50 ml Tetrahydrofuran zu und rührt während 15 Stunden bei 20-25°C aus. Die Reaktionslösung wird klärfiltriert, mit 1 g Chloranil versetzt, mit Ammoniaklösung entfärbt und zur Trockene eingeengt. Der Rückstand wird in 150 ml Salzsäure (30 %) aufgenommen und bei 80-85°C mit Toluol behandelt. Die wässrige Phase wird mit wässriger Natriumhydroxidlösung auf pH 12 gestellt und mit Toluol behandelt, worauf die Toluolphase nach Entfernung des Toluols 9,1 g einer Fluoranverbindung der Formel

(16) 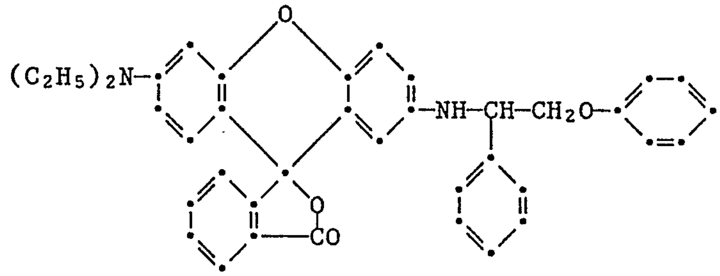

in Form eines diastereomeren Gemisches ergibt. Durch Umkristallisation aus Toluol erhält man 1,7 g der Komponente A Smp. 193-194,5°C. Komponente A entwickelt auf säuremodifiziertem Ton sofort eine graue Färbung, mit Phenolharz eine grün-graue Färbung mit sehr guter Lichtechtheit.

Aus dem Filtrat der Umkristallisationsstufe gewinnt man durch Säulenchromatographie an Kieselgel mit Toluol und Essigsäureethylester (9:1) 1,6 g der Komponente B, Smp. 160-161°C. Letztere entwickelt auf säuremodifiziertem Ton und auf Phenolharz eine lichtechte graue Färbung.

Beispiel 13: Herstellung eines druckempfindlichen Kopierpapiers

Eine Lösung von 3 g der Fluoranverbindung der Formel (11) (Beispiel 1) in 80 g Diisopropylnaphthalin und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive graue Kopie. Dieses Kopiermaterial besitzt eine ausgezeichnete Sublimierechtheit.

Beispiel 14:

1 g der Komponente A der Fluoranverbindung der Formel (11) gemäss Beispiel 1 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1:1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich sofort auf dem mit dem Farbbildner beschichteten Blatt eine intensive graue Farbe.

Beispiel 15: Herstellung eines wärmeempfindlichen Aufzeichnungsmaterials

In einer Kugelmühle werden 32 g 4,4'Isopropylidendiphenol (Bisphenol A), 3,8 g Distearylamid des Ethylendiamins, 39 g Kaolin, 20 g eines zu 88 % hydrolysierten Polyvinylalkohols und 500 ml Wasser gemahlen bis die Teilchengrösse ca. 5 µm beträgt. In einer zweiten Kugelmühle werden 6 g der Fluoranverbindung gemäss Beispiel 1, 3 g eines zu 88 % hydrolysierten Polyvinylalkohols und 60 ml Wasser zu einer Teilchengrösse von ca. 3 µm gemahlen.

Die beiden Dispersionen werden zusammengegeben und mit einem Trockenauftragsgewicht von 5,5 g/m² auf ein Papier gestrichen. Durch Berührung des Papiers mit einem erhitzten Metallstift wird eine intensive graue Farbe erhalten, die eine ausgezeichnete Lichtechtheit hat.

**Patentansprüche**

1. 2-Aralkylamino-Fluoranverbindungen der Formel

(1) 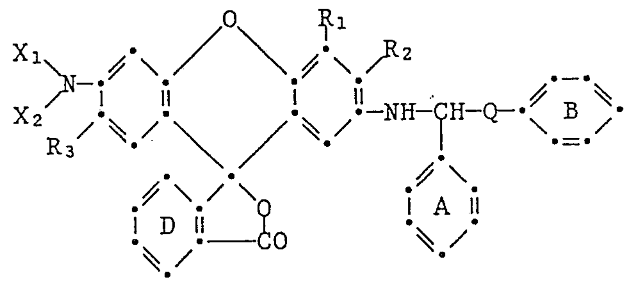

worin
Q -$CH_2$-, -$CH_2$-0- oder -CO-,
$R_1$, $R_2$ und $R_3$, unabhängig voneinander, je Wasserstoff, Halogen, Niederalkyl oder Niederalkoxy,
$X_1$ und $X_2$, unabhängig voneinander, je Wasserstoff, unsubstituiertes oder durch Halogen, Hydroxy, Cyano, Tetrahydrofuryl oder Niederalkoxy substituiertes Alkyl mit höchstens 12 Kohlenstoffatomen, Cycloalkyl oder unsubstituiertes oder durch Halogen, Cyano, Nitro, Niederalkyl, Niederalkoxy oder Niederalkoxycarbonyl substituiertes Benzyl oder Phenyl oder
$X_1$ und $X_2$ zusammen mit dem sie verbindenden Stickstoffatom einen fünf-oder sechsgliedrigen, heterocyclischen Rest bedeuten und worin
die Benzolringe A und B, unabhängig voneinander, unsubstituiert oder durch Halogen, Cyano, Niederalkyl, Niederalkoxy, Niederalkylthio, Niederalkoxycarbonyl oder Trifluormethyl substituiert sind und der Ring D unsubstituiert oder durch Halogen, Nitro, Niederalkyl, Niederalkoxy, Niederalkylthio, Amino, Mononiederalkylamino oder Diniederalkylamino substituiert ist.

2. Fluoranverbindungen gemäss Anspruch 1, dadurch gekennzeichnet, dass in Formel (1) $R_1$, $R_2$ und $R_3$, unabhängig voneinander, je Wasserstoff, Methyl, Methoxy oder Chlor bedeuten.

3. Fluoranverbindungen gemäss einem der Ansprüche 1 und 2, dadurch gekennzeichnet, dass in Formel (1) $X_1$ und $X_2$, unabhängig voneinander, Niederalkyl, Cyclohexyl, Tolyl, Benzyl oder Cyanoniederalkyl oder -$NX_1X_2$ Pyrrolidinyl oder Tetrahydrofurfurylamino oder N-Niederalkyl-N-tetrahydrofurfurylamino bedeuten.

4. Fluoranverbindungen gemäss einem der Ansprüche 1 bis 3, dadurch gekennzeichnet, dass in Formel (1) die Benzolringe A und B, unabhängig voneinander, je unsubstituiert oder durch Methyl, Chlor, Cyano, Trifluormethyl, Methoxy oder Carbomethoxy substituiert sind.

5. Fluoranverbindungen gemäss Anspruch 1, dadurch gekennzeichnet, dass sie der Formel

(2) 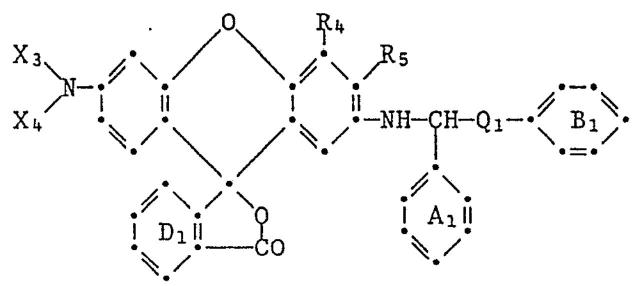

entsprechen, worin
$Q_1$ -$CH_2$- oder -CO-,
$R_4$ und $R_5$, unabhängig voneinander, je Wasserstoff, Halogen oder Niederalkyl,
$X_3$ $C_1$-$C_6$-Alkyl, $C_5$-$C_6$-Cycloalkyl, Benzyl, Phenyl oder durch Halogen, $C_1$-$C_4$-Alkyl oder $C_1$-$C_4$-Alkoxy substituiertes Phenyl, $X_4$ $C_1$-$C_6$-Alkyl oder Benzyl oder -$NX_3X_4$ Pyrrolidinyl, Piperidinyl, Morpholinyl, Tetrahydrofurfurylamino oder N-Niederalkyl-N-tetrafurfurylamino
bedeuten und worin
die Benzolringe $A_1$ und $B_1$, unabhängig voneinander, je unsubstituiert oder durch Halogen, Niederalkyl oder Niederalkoxy, substituiert sind und der Ring $D_1$ unsubstituiert oder durch Halogen substituiert ist.

6. Fluoranverbindungen gemäss Anspruch 5, dadurch gekennzeichnet, dass in Formel (2) $X_3$ und $X_4$ $C_1$-$C_4$-Alkyl, $R_4$ und $R_5$ Wasserstoff, Methyl oder Chlor und die Ringe $A_1$, $B_1$ und $D_1$ unsubstituiert sind.

7. Fluoranverbindungen gemäss Anspruch 1, dadurch gekennzeichnet, dass sie der Formel

(3) 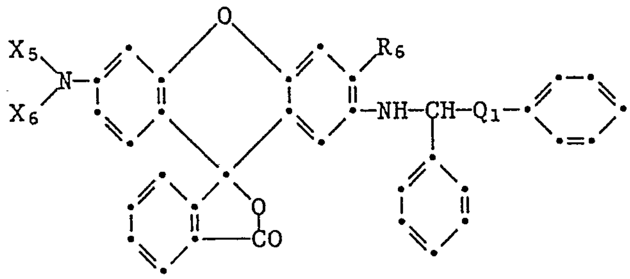

entsprechen,
worin $Q_1$ -$CH_2$- oder -CO-,
$R_6$ Wasserstoff oder Methyl,
$X_5$ $C_1$-$C_4$-Alkyl, Cyclohexyl oder Tolyl
und $X_6$ $C_1$-$C_4$-Alkyl bedeuten.

8. Verfahren zur Herstellung von 2-Aralkylamino-Fluoranverbindungen der Formel (1) gemäss Anspruch 1, dadurch gekennzeichnet, dass man eine 2-Amino-Fluoranverbindung der Formel

(4) 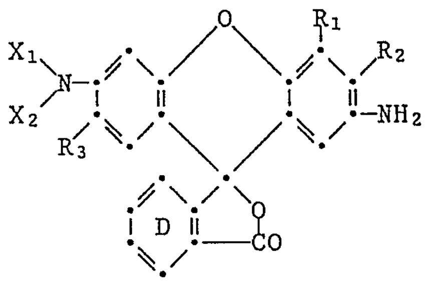

worin $R_1$, $R_2$, $R_3$, $X_1$, $X_2$ und D die in Anspruch 1 angegebene Bedeutung haben, mit einem Aralkylierungsmittel der Formel

(5) 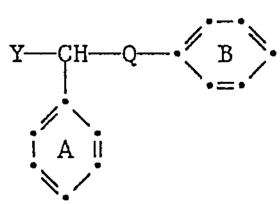

worin A, B und Q die in Anspruch 1 angegebene Bedeutung haben und
Y Halogen, Alkylsulfonyloxy oder Arylsulfonyloxy bedeutet, umsetzt.

9. Abänderung des Verfahrens gemäss Anspruch 8, dadurch gekennzeichnet, dass man eine 2-Amino-Fluoranverbindung der Formel (4) mit einer Verbindung der Formel

(8) 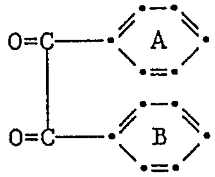

worin A und B die in Anspruch 8 angegebene Bedeutung haben, umsetzt und das Umsetzungsprodukt der Formel

(9)

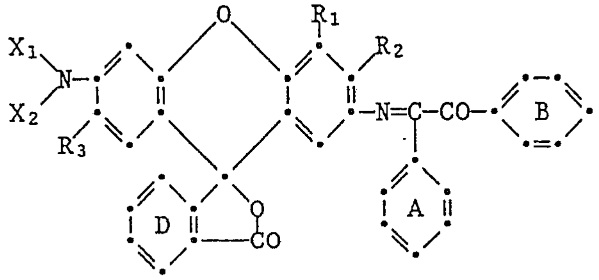

worin A, B, D, $R_1$, $R_2$, $R_3$, $X_1$ und $X_2$ die in Anspruch 8 angegebene Bedeutung haben, reduziert.

10. Abänderung des Verfahrens gemäss Anspruch 8, dadurch gekennzeichnet, dass man eine 2-Amino-Fluoranverbindung der Formel (4) mit einer Carbinolverbindung der Formel

(10)

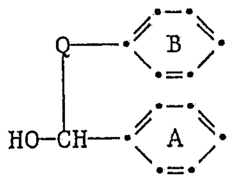

worin A, B und Q die im Anspruch 8 angegebene Bedeutung haben, umsetzt.

11. Verwendung von 2-Aralkylamino-Fluoranverbindungen der in einem der Ansprüche 1 bis 7 angegebenen Formel als Farbbildner in einem druckempfindlichen oder wärmeempfindlichen Aufzeichnungsmaterial.

12. Druck- oder wärmeempfindliches Aufzeichnungsmaterial, dadurch gekennzeichnet, dass es in seinem Farbreaktantensystem als Farbbildner mindestens eine Fluoranverbindung der in einem der Ansprüche 1 bis 7 angegebenen Formel enthält.

13. Druckempfindliches Aufzeichnungsmaterial gemäss Anspruch 12, dadurch gekennzeichnet, dass es die Fluoranverbindung, gelöst in einem organischen Lösungsmittel, und mindestens einen festen Elektronenakzeptor enthält.

14. Druckempfindliches Aufzeichnungsmaterial gemäss einem der Ansprüche 12 und 13, dadurch gekennzeichnet, dass die Fluoranbindung in Mikrokapseln eingekapselt ist.

15. Druckempfindliches Aufzeichnungsmaterial gemäss Anspruch 14, dadurch gekennzeichnet, dass die eingekapselte Fluoranverbindung in Form einer Schicht auf der Rückseite eines Uebertragungsblattes und der Elektronenakzeptor in Form einer Schicht auf der Vorderseite des Empfangsblattes vorhanden sind.

16.Druckempfindliches Aufzeichnungsmaterial gemäss einem der Ansprüche 12 bis 15, dadurch gekennzeichnet, dass die Fluoranverbindung gemeinsam mit einem oder mehreren anderen Farbbildnern enthalten ist.

17. Wärmeempfindliches Aufzeichnungsmaterial gemäss Anspruch 12, dadurch gekennzeichnet, dass es in mindestens einer Schicht mindestens eine Fluoranverbindung der in einem der Ansprüche 1 bis 7 angegebenen Formel, einen Elektronenakzeptor und gegebenenfalls ein Bindemittel und/oder Wachs enthält.

Europäisches Patentamt

# EUROPÄISCHER RECHERCHENBERICHT

Nummer der Anmeldung

EP 89810135.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | EP - A1 - 0 115 821 (HODOGAYA CHEMICAL CO.)<br>* Anspruch 1; Seite 1, Zeilen 1-5 *<br>-- | 1,5,8, 11-17 | C 07 D 493/10<br>G 03 C 1/733<br>B 41 M 5/12<br>B 41 M 5/14<br>B 41 M 5/22 |
| A | DE - C2 - 2 424 935 (SHIN NISSO KAKO CO.)<br>* Ansprüche 1,17; Seite 14, Verbindungen 10,11 *<br>-- | 1,5,8, 11-17 | //(C 07 D 493/10,<br>C 07 D 311:00,<br>C 07 D 307:00) |
| A | CHEMICAL ABSTRACTS, Band 101, Nr. 8, 20. August 1984, Columbus, Ohio, USA<br>SUMITOMO CHEMICAL CO. "Imaging paper" Seite 544, Spalte 2, Zusammenfassung-Nr. 63 724p<br>& Jpn. Kokai Tokkyo Koho JP 58 4 088 [83 45 088]<br>-- | 1,5,8, 11-17 | |
| | | | RECHERCHIERTE SACHGEBIETE (Int. Cl.4) |
| | | | C 07 D 493/00<br>B 41 M 5/00 |
| A | CHEMICAL ABSTRACTS, Band 101, Nr. 4, 23. Juli 1984, Columbus, Ohio, USA<br>SUMITOMO CHEMICAL CO. "Imaging sheets" Seite 498, Spalte 2, Zusammenfassung-Nr. 31 233m<br>& Jpn. Kokai Tokkyo Koho JP 58 56 888 [83 56 888]<br>---- | 1,5,8, 11-17 | |

Der vorliegende Recherchenbericht wurde für alle Patentansprüche erstellt.

| Recherchenort | Abschlußdatum der Recherche | Prüfer |
|---|---|---|
| WIEN | 17-05-1989 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82